# EUROPÄISCHE PATENTSCHRIFT

(11) **EP 3 000 500 B1**
(45) Veröffentlichungstag und Bekanntmachung des Hinweises auf die Patenterteilung: **27.05.2020**
(21) Anmeldenummer: 15002498.2
(22) Anmeldetag: 24.08.2015
(51) Int. Cl.: A61M 16/06

(54) **MASKENKISSEN FÜR EINE VOLLGESICHTSMASKE**
MASK CUSHION FOR A FULL FACE MASK
BULLE DE MASQUE POUR UN MASQUE COMPLET DU VISAGE

(30) Priorität: 23.09.2014 DE 102014013796
(43) Veröffentlichungstag der Anmeldung: 30.03.2016
(73) Patentinhaber: Löwenstein Medical Technology S.A., 2557 Luxembourg (LU)
(72) Erfinder: Eifler, Martin, 25348 Glückstadt (DE)
(74) Vertreter: Marx, Thomas

(56) Entgegenhaltungen:
- EP-A2- 2 737 921
- WO-A1-2010/111749
- DE-A1- 10 002 571
- US-A1- 2014 216 462

## Beschreibung

Masken werden im Bereich der Beatmung und der Schlaftherapie eingesetzt, wobei ein Patient mittels einer Flow- oder Druckquelle mit Atemgas versorgt wird. Die Maske stellt die Schnittstelle zwischen Patient und Gerät dar. Sie besteht aus einem Maskenkörper mit einem Maskenkissen und wird meistens mittels einer Bänderung und einer zusätzlichen Stirnabstützung am Kopf des Patienten fixiert. Der Maskenkörper besteht in der Regel aus einem relativ steifen Kunststoff und das Kissen, das am Gesicht des Patienten anliegt, besteht aus einem relativ dünnen, weichen Material, vorzugsweise aus einem hautfreundlichen Silikon, einem thermoplastischen Elastomer (TPE) oder aus Polyurethan und besitzt in der Regel eine Dichtlippe, um eine ausreichende Abdichtung zu gewährleisten und Leckagen zu verhindern. Diese Masken können als Nasalmasken oder als Vollgesichtsmasken (oder auch Mund-Nasen-Masken) ausgeführt sein.

Herkömmliche Masken gibt es üblicherweise in den Größen S, M und L, um möglichst viele verschiedene Gesichts- bzw. Nasenbeschaffenheiten der Patienten zu berücksichtigen und einen möglichst hohen Trage- und Passkomfort und eine hohe Dichtigkeit zu erzielen.

Derartig bekannte Lösungen haben zur Folge, dass die Anzahl anzubietender Masken groß ausfallen muss, um alle verschiedenen Gesichtsformen und Größen abzudecken. Im Bereich der Nasalmasken ist eine "Universalmaske" in einer Einheitsgröße bekannt. Vollgesichtsmasken in nur einer Größe die für eine größere Anzahl von Gesichtsformen und -größen geeignet sind, sind nicht bekannt. Dies ist darin begründet, dass die Variabilität der Abstände zwischen Nase und Unterlippe oder Kinn groß ist. Zudem müssen noch unterschiedlich große Nasen berücksichtigt werden.

Das Maskenkissen der US 2014/021 64 62 A1 hat einen dreitilgen Aufbau bestehend aus einem "Rigid clip", einem "Soft clip" und dem "Foam" (Schaum). Dabei liegt der Schaum dem Gesicht als Dichtbereich an und bietet durch seine ausgeprägte Materialdicke eine komfortable Abdichtung.

Die vorliegende Erfindung stellt sich zur Aufgabe ein Maskenkissen für Vollgesichtsmasken zu entwickeln, welches eine große Anpassungsfähigkeit an verschieden große und verscheiden geformte Gesichts-, Nasen- und Kinnpartien der Patienten aufweist. Patienten können diverse verschiedene Gesichtsformen und Größen mit verschiedenen Nasenformen mit hohen oder niedrigen Nasenrückenregionen, schmale oder breite sowie lange oder kurze Nasen haben. Der Mund- und Kinnbereich kann ebenfalls sehr unterschiedlich groß und unterschiedlich geformt sein, deshalb soll das Maskenkissen in der Lage sein, sich allen möglichen Gesichtsformen und -größen anzupassen und die gewünschte Passform und Dichtigkeit zu erzielen.

Die Aufgabe wird gelöst durch ein Maskenkissen nach Anspruch 1.

Die Erfindung ist auch dadurch gekennzeichnet, dass die erste Höhe (H1) größer als die zweite Höhe (H2) ist und die zweite Höhe größer als die dritte Höhe (H3) ist.

Die Erfindung ist auch dadurch gekennzeichnet, dass der Verformungsbereich unten an der Basis (H1) 25 mm +/- 3 mm hoch ist und/ oder oben im Nasenrückenbereich (H2) 20 mm +/- 2,5 mm hoch ist und/oder seitlich im Nebennasenbereich (H3) 11 mm +/- 2 mm.

Die Erfindung ist auch dadurch gekennzeichnet, dass das Maskenkissen aus einem elastomeren Kunststoff hergestellt ist und zumindest zwei unterschiedliche Wandstärken aufweist.

Die Erfindung ist auch dadurch gekennzeichnet, dass die Wandstärke im Verformungsbereich unten an der Basis (W2) und oben im Nasenrückenbereich (W1) dünner ist als seitlich im Nebennasenbereich (W3).

Die Erfindung ist auch dadurch gekennzeichnet, dass die Wandstärke im Verformungsbereich im Nasenrückenbereich (W1) dünner ist als die Wandstärke im Verformungsbereich an der Basis (W2) und dass die Wandstärke (W2) dünner ist als seitlich im Nebennasenbereich (W3).

Die Erfindung ist auch dadurch gekennzeichnet, dass die Wandstärke (W3) zumindest doppelt so dick ist wie die Wandstärke (W2).

Die Erfindung ist auch dadurch gekennzeichnet, dass die Länge (L) 105 mm +/- 6 mm und die Breite (B2) 90 mm +/- 4 mm ist.

Die Erfindung ist auch dadurch gekennzeichnet, dass die Breite im Nasenrückenbereich außen (B1) 3x, bevorzugt 4x, breiter ist als im Innenausschnitt des Nasenbereiches (B3).

Die Erfindung ist auch dadurch gekennzeichnet, dass die Breite im Nasenbereich außen (B1) 35 mm +/- 3 mm ist und im Innenausschnitt des Nasenbereiches (B3) 8 mm +/- 2 mm.

Die Erfindung ist auch dadurch gekennzeichnet, dass die Vollgesichtsmaske eine stufenlos verstellbare Stirnstütze aufweist.

Die Erfindung ist auch dadurch gekennzeichnet, dass die Stirnstütze ein Federelement aufweist.

Die Erfindung ist auch dadurch gekennzeichnet, dass das Federelement ein Elastomer ist.

Die Erfindung ist auch dadurch gekennzeichnet, dass die stufenlos verstellbare Stirnstütze durch die Anzugkraft der Kopf-Bänderung eingestellt wird.

Die Erfindung ist auch dadurch gekennzeichnet, dass im Nasenrückenbereich die Wandstärke (W1) von ≤ 0,6 mm ist.

Die Erfindung ist auch dadurch gekennzeichnet, dass im Nasenrückenbereich die Wandstärke (W1) im Bereich 0,2 - 0,5 mm ist.

Die Erfindung ist auch dadurch gekennzeichnet, dass der Seitenbereich eine Wandstärke (W3) im Bereich 1,2-2,9 mm aufweist.

Die Erfindung ist auch dadurch gekennzeichnet, dass der Seitenbereich eine Wandstärke (W3) im Bereich 1,5-2,5 mm, bevorzugt 2,1 mm +/- 2 mm aufweist.

Die Erfindung ist auch dadurch gekennzeichnet, dass die Wandstärke W2 von der Basis hin zur Wandstärke W4 der Dichtlippe kontinuierlich abnimmt.

### Figurenbeschreibung

Fig.1a: Maskenkissen in Draufsicht
Fig.1b: Maskenkissen in Seitenansicht
Fig.1c: Maskenkissen von oben
Fig.2a: vertikale Schnittdarstellung entlang der Ebene x/x
Fig.2b: horizontale Schnittdarstellung entlang der Ebene y/y
Fig.2c: horizontale Schnittdarstellung entlang der Ebene z/z
Fig.3: Vergleich Erfindung zum Stand der Technik
Fig.4: Vergleich Erfindung zum Stand der Technik in verschiedenen Ansichten
Fig.5: Vergleich Erfindung zum Stand der Technik Schnittdarstellungen

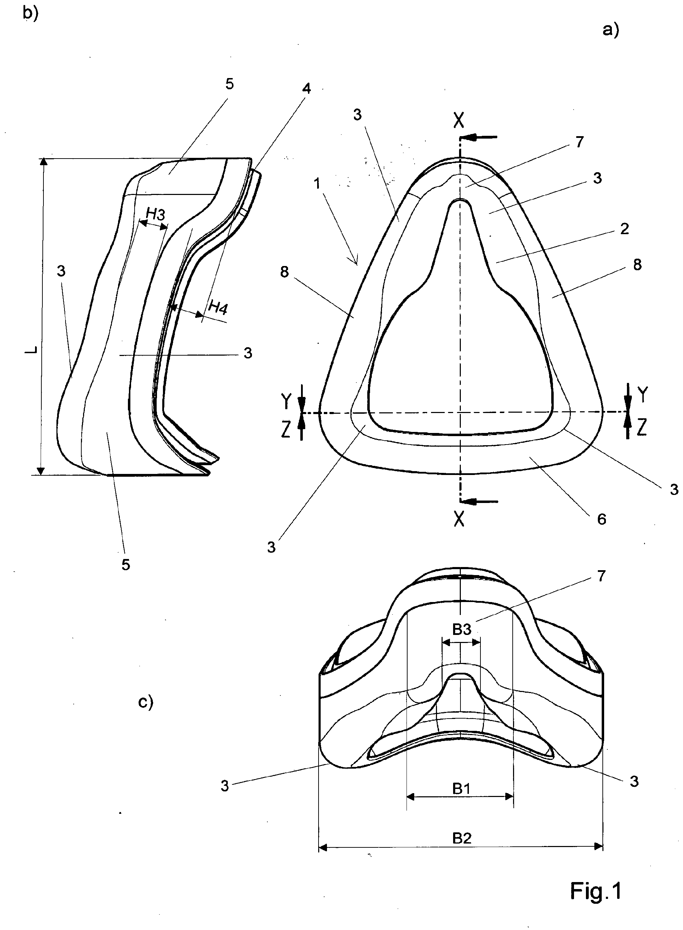

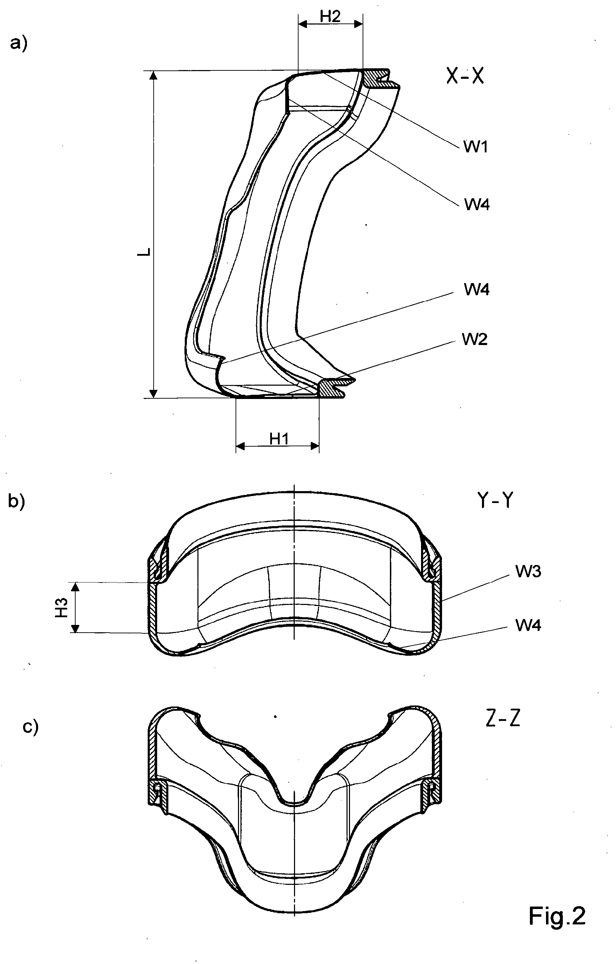

Die Figur 1 zeigt das Maskenkissen in der Draufsicht. Eingezeichnet ist eine vertikale Schnittebene x und eine horizontale Schnittebene y/z. Die Schnitte sind in Figur 2 veranschaulicht. Das Kissen weist einen Nasenrückenbereich (7), Seitenbereiche (8), den Basisbereich (6) und den Dichtbereich (3) zum Patienten auf. Die Seitenbereiche (8) erstrecken sich ausgehend vom Kontaktbereich zum Maskenkörper (4) im Wesentlichen vertikal nach oben. Der Dichtbereich (3) mündet in einer Einzeldichtlippe (2), die den anderen Bereichen angeformt ist und innen eine Öffnung freigibt, die der Einführung von zumindest der Nase und Mund des Patienten dient. Der Dichtbereich (3) erstreckt sich ausgehend von den Seitenbereichen und dem Basisbereich sowie dem Nasenrückenbereich zur Öffnung hin, wobei sich der Dichtbereich (3) zumindest teilweise horizontal zum Seitenbereich (8) erstreckt. Ein Verformungsbereich (5) erstreckt sich zwischen dem Kontaktbereich (4) und dem Dichtbereich (3). Im Seitenbereich (8) ist der Verformungsbereich (5) jedoch durch die Verwendung einer höheren Wandstärke eher ein Stützbereich. Der Kontaktbereich (4) kann im Sinne der Erfindung, wie hier ausgeführt, als mechanische Verbindung zu einem Hartteil des Maskenkörpers ausgeführt sein oder auch direkt in den Hartteil des Maskenkörpers übergeleitet sein, beispielsweise als 2K-Verbindung, oder als Klebung ausgeführt sein.

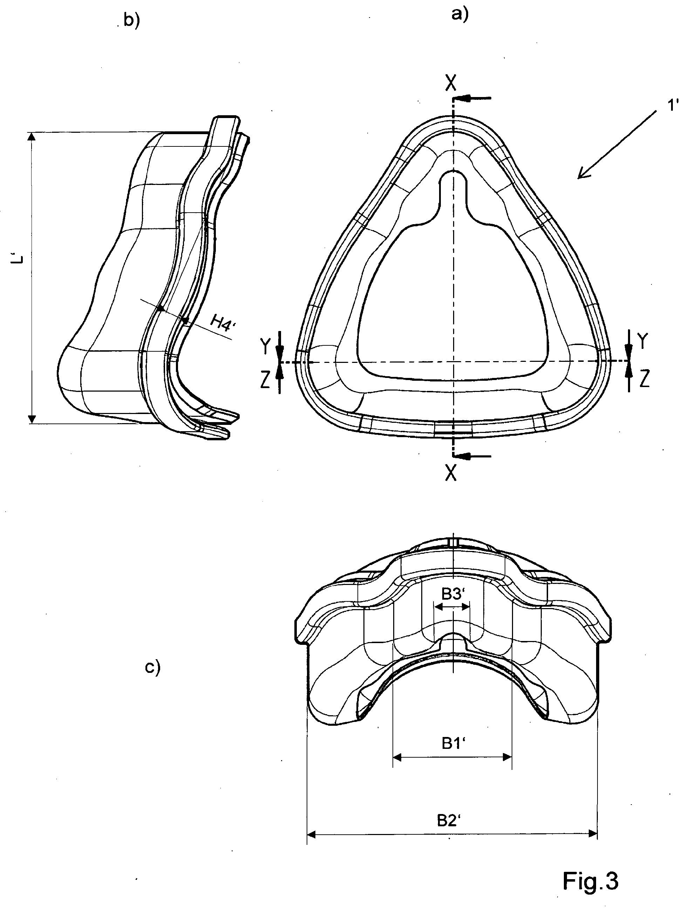

Die Figur 1b zeigt das Kissen in der Seitenansicht. Der Kontaktbereich (4) weist eine starke Einbuchtung mit der Höhe (H4) auf. Im Vergleich zur Seitenansicht des Kissens aus dem Stand der Technik Fig. 3 ist deutlich zu erkennen, dass H4 im Stand der Technik deutlich weniger ausgeprägt ist. Die starke Einbuchtung mit der Höhe (H4) des erfindungsgemäßen Maskenkissens dient dazu, den Verformungsbereich (5) im Seitenbereich (8) des Kissens möglichst kurz und stabil auszuführen. Die maximale Länge (L) des Kissens ist 105 mm +/- 6 mm und die Breite (B2) 90 mm +/-4 mm.

Figur 1c zeigt das Kissen in der Ansicht von oben. Die Breite des Nasenrückenbereiches (B1) wurde breiter als bei bekannten Masken gewählt, um eine optimale Passform und Dichtigkeit auch bei breiteren und auch flachen Nasenformen zu gewährleisten. Durch die gewählte Geometrie sitzt das Kissen tiefer auf der Nase des Anwenders. Ein hoher Nasenrücken taucht bei der gewählten Kontur tiefer in das Kissen ein. Die Breite im Nasenrückenbereich außen (B1) ist zumindest 3x, bevorzugt 4x, breiter als im Innenausschnitt des Nasenbereiches (B3). B2 stellt die maximale Breite des Kissens dar.

Die Figur 2a zeigt das Kissen in der Ebene x geschnitten. Im Nasenrückenbereich (7) ist der Patient am empfindlichsten, daher wird hier eine sehr dünne Wand (W1) von ≤ 0,6 mm, bevorzugt 0,2 - 0,5 mm, besonders bevorzugt 0,25 mm gewählt. Dieser Bereich lässt sich leicht verformen und der Nasenform anpassen. Die Wand der Dichtlippe (W4) kann dieselbe Dicke aufweisen wie die Wand W1 oder auch dicker sein. Dann wäre die Dicke W1 0,4 mm und W4 0,5 mm.

Der Basisbereich (6), mit der Wandstärke (W2), ist im Gegensatz zu den Seitenbereichen (8), mit den Wandstärken (W3), ebenfalls dünn ausgeführt, um eine möglichst große Flexibilität für den Kinnbereich zu erhalten. Die Wandstärke (W2) beträgt im Bereich 0,5 - 1,2 mm, bevorzugt 1,0 mm. Da der Patient an den Kontaktstellen im Seitenbereich (8) des Kissens am unempfindlichsten ist, können die Wandstärken (W3) dieses Bereiches dicker ausgeführt sein und sorgen so für die Steifigkeit und Stützkraft des Kissens. Die Wandstärke (W3) beträgt in diesem Bereich 1,2 - 2,9 mm, bevorzugt 1,5-2,5 mm, besonders bevorzugt 2,1 mm. Die Dichtlippe ist als eine Einzeldichtlippe ausgeführt. Die Wandstärke W4 der Dichtlippe wurde auf dem kompletten Umfang mit einer annähernd gleichbleibenden, dünnen Wandstärke (W4) von 0,2 - 0,75 mm, bevorzugt 0,5 mm, ausgebildet, die dem Patienten einen angenehmen Tragekomfort ermöglicht.

Die Höhe (H2), gemessen für den Bereich der Wandstärke (W1) vom Kontaktbereich (4) bis zum Radius, im Nasenrückenbereich beträgt 18 - 25 mm, bevorzugt 20 - 25 mm, besonders bevorzugt 20 mm +/-2,5 mm und ist höher als bei Masken aus dem Stand der Technik. Dadurch wird ein weiter Bereich mit guten Dichteigenschaften für unterschiedliche Nasenrücken eröffnet.
Die Höhe (H1) des Basisbereiches (6) ist höher als bei handelsüblichen Maskenkissen und beträgt im Bereich 23 - 28 mm, bevorzugt 25 mm +/- 3 mm, besonders bevorzugt 25 mm +/- 2 mm.

Aus Fig. 1 und 2 wird deutlich, dass die angegebenen Höhen H1, H2, H3 sich auf den Verformungsbereich (5) beziehen. Dieser erstreckt sich zwischen der Außenkante des Kontaktbereiches (4) und dem Übergang zum Dichtbereich (3). Genauer ausgehend von der Außenkante des Kontaktbereiches (4), also oberhalb der verdickten Zone, bis zum Beginn des Radius der in den Dichtbereich (3) übergeht.

Die Figur 2b zeigt das Kissen in der Ebene y geschnitten. Die Höhe (H3) des Seitenbereiches (8) ist geringer als bei handelsüblichen Maskenkissen und beträgt im Bereich 8 - 13 mm, bevorzugt 11 mm +/-2 mm. Der Verformungsbereich (5) unten an der Basis (6) weist eine erste Höhe (H1) auf, oben im Nasenrückenbereich (7) eine zweite Höhe (H2) auf und seitlich im Nebennasenbereich (8) eine dritte Höhe (H3) auf, wobei die erste Höhe (H1) größer als die zweite Höhe (H2) ist und die zweite Höhe größer als die dritte Höhe (H3) ist und wobei die erste Höhe (H1) zumindest doppelt so hoch ist, wie die dritte Höhe (H3).

Die Figur 2c zeigt das Kissen in der Ebene z geschnitten.
In der Figur 3 werden die Unterscheide des erfindungsmäßigen Maskenkissens (M) zu Kissen aus dem Stand der Technik (M')in der Perspektivdarstellung deutlich. Insbesondere ist gut erkennbar, dass die Höhe H4' im SdT geringer ist und die Breite B1' größer ist. Erfindungsgemäß ist H4 im Bereich 10 - 14 mm, bevorzugt über 11 mm. Im SdT ist H4'im Bereich unter 8 mm.

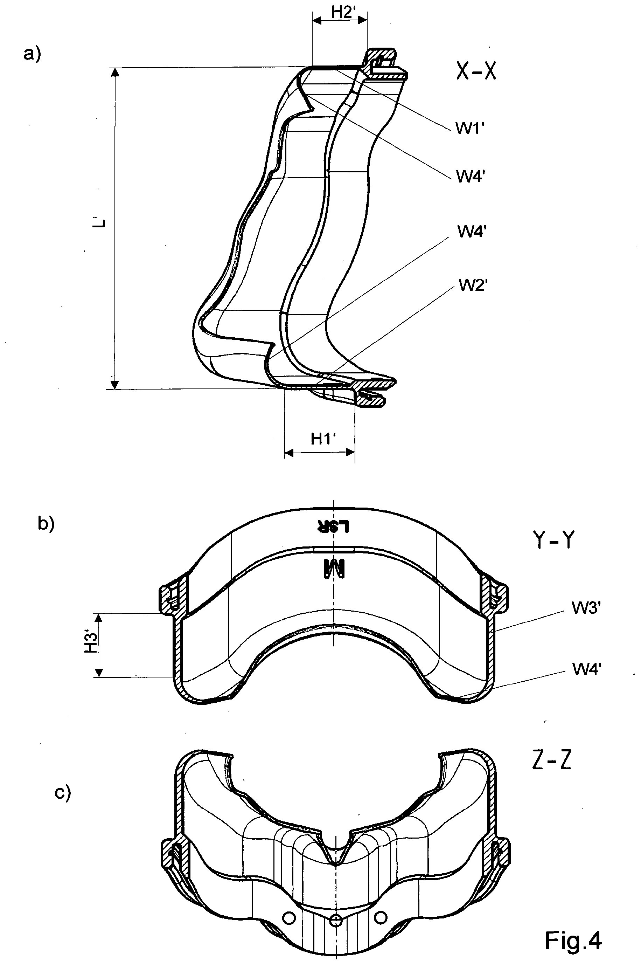

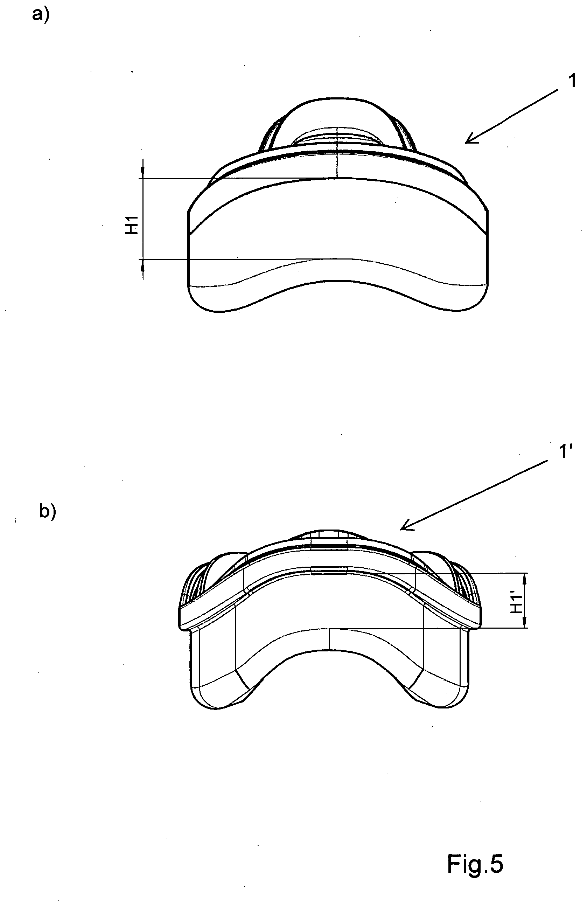

Die Figuren 4 und 5 zeigen Unterscheide des erfindungsmäßigen Maskenkissens (M) zu Kissen aus dem Stand der Technik (M')

Eine Gegenüberstellung des Maskenkissens (M) zu den Kissen aus dem Stand der Technik (M') macht den Unterscheid der Erfindung deutlich.

Die maßgeblichen Merkmale des erfindungsgemäßen Kissens sind der sehr große Verformbereich des Nasenrückenbereiches (1), der durch die geeignete Wahl der Wandstärke (W1) und der Breite (B1), im Verhältnis zur Breite B3 und der Höhe (H2) erzielt wird. Dieser Bereich ist ausschlaggebend für die Anpassungsfähigkeit an verschieden große und verschieden geformte Nasenpartien der Patienten. Zudem sind bei dem erfindungsgemäßen Kissen die Wandstärke (W2) an der Basis, die Höhe an der Basis (H1), sowie die Wandstärke (W3) an der Seite, die Höhe an der Seite (H3) ausschlaggebend für eine große Anpassbarkeit an der Basis und eine hohe Stützfunktion im Seitenbereich (8).
Die Wandstärke (W2') ist 1 mm; (W1') 0,5 mm; (W3') 1,5 mm; W4'0,5 mm. Die Wandstärke (W3') ist also nicht doppelt so dick ist wie die Wandstärke (W2').

Aus dem Vergleich der Erfindung mit dem Stand der Technik (siehe Figuren 3 bis 5) wird deutlich, dass bei dem erfindungsgemäßen Kissen H1 größer ist als H1'; H3 kleiner ist, als H3'; H4 größer ist, als H4'; W2 kleiner ist als W2'. Seitlich (8) ist das erfindungsgemäße Kissen etwa um 1/3 flacher, bezogen auf H3, als Kissen aus dem Stand der Technik, dadurch wird eine stabile Kante zur besseren Auflage seitlich der Nase erzielt.
Dafür ist notwendig dass Maskenkörper in dem Seitenbereich höher ist, was zu einer ausgeprägten Höhe H4 (mit etwa 12 mm) führt.

## Patentansprüche

1. Maskenkissen (1) für eine Vollgesichtsmaske (10) mit lediglich einer Dichtlippe (2), einem zum Patientengesicht weisenden Dichtbereich (3) der in der Dichtlippe mündet, einem Kontaktbereich (4) zum Maskenkörper (10) und einem Verformungsbereich (5) der Bich zwischen dem Kontaktbereich (4) und dem Dichtbereich (3) erstreckt, wobei der Verformungsbereich (5) unten an der Basis (6) eine erste Höhe (H1) aufweist, oben im Nasenrückenbereich (7) eine zweite Höhe (H2) aufweist und seitlich im Nebennasenbereich (8) eine dritte Höhe (H3) aufweist, wobei die erste Höhe (H1) zumindest doppelt so hoch ist, wie die dritte Höhe (H3), wobei das Maskenkissen (1) eine maximale Länge (L) und eine maximale Breite (B2) und eine Breite (B1) im Nasenrückenbereich außen und eine Breite (B3) im Innenausschnitt des Nasenbereiches aufweist **dadurch gekennzeichnet, dass** die maximale Länge (L) 105 mm +/- 6 mm und die maximale Breite (B2) 90 mm +/- 4 mm ist und wobei die Breite im Nasenrückenbereich außen (B1) zumindest 3x, bevorzugt 4x, breiter ist als die Breite im Innenausschnitt des Nasenbereiches (B3) .

2. Maskenkissen (1) für eine Vollgesichtsmaske (10) nach Anspruch 1 **dadurch gekennzeichnet, dass** die erste Höhe (H1) größer als die zweite Höhe (H2) ist und die zweite Höhe größer als die dritte Höhe (H3) ist.

3. Maskenkissen (1) für eine Vollgesichtsmaske (10) nach zumindest einem der vorhergehenden Ansprüche **dadurch gekennzeichnet, dass** der Verformungsbereich (5) unten an der Basis (6) (H1) 25 mm +/- 3 mm hoch ist und/oder oben im Nasenrückenbereich (7) (H2) 20 mm +/- 2,5 mm hoch ist und/oder seitlich im Nebennasenbereich (8) (H3) 11 mm +/- 2 mm.

4. Maskenkissen (1) für eine vollgesichtsmaske (10) nach zumindest einem der vorhergehenden Ansprüche **dadurch gekennzeichnet, dass** das Maskenkissen aus einem elastomeren Kunststoff hergestellt ist und zumindest zwei unterschiedliche Wandstärken (9) aufweist.

5. Maskenkissen (1) für eine Vollgesichtsmaske (10) nach zumindest einem der vorhergehenden Ansprüche **dadurch gekennzeichnet, dass** die Wandstärke (9) im Verformungsbereich (5) unten an der Basis (W2) und oben im Nasenrückenbereich (W1) dünner ist als seitlich im Nebennasenbereich (W3).

6. Maskenkissen (1) für eine Vollgesichtsmaske (10) nach zumindest einem der vorhergehenden Ansprüche **dadurch gekennzeichnet, dass** die Wandstärke (9) im Verformungsbereich im Nasenrückenbereich . (W1) dünner ist als die Wandstärke im Verformungsbereich an der Basis (W2) und dass die Wandstärke (W2) dünner ist als seitlich im Nebennasenbereich (W3).

7. Maskenkissen (1) für eine Vollgesichtsmaske (10) nach zumindest einem der vorhergehenden Ansprüche **dadurch gekennzeichnet, dass** die Wandstärke (W3) zumindest doppelt so dick ist wie die Wandstärke (W2).

8. Maskenkissen (1) für eine Vollgesichtsmaske (10) nach zumindest einem der vorhergehenden Ansprüche **dadurch gekennzeichnet, dass** die Breite im Nasenbereich außen (B1) 35 mm +/- 3 mm ist und im Innenausschnitt des Nasenbereiches (B3) 8 mm +/- 2 mm.

9. Maskenkissen (1) für eine Vollgesichtsmaske (10) nach zumindest einem der vorhergehenden Ansprüche **dadurch gekennzeichnet, dass** im Nasenrückenbereich (7) die Wandstärke (W1) von ≤ 0,6 mm ist.

10. Maskenkissen (1) für eine Vollgesichtsmaske (10) nach zumindest einem der vorhergehenden Ansprüche **dadurch gekennzeichnet, dass** im Nasenrückenbereich (7) die Wandstärke (W1) im Bereich 0,2 - 0,5 mm ist.

11. Maskenkissen (1) für eine Vollgesichtsmaske (10) nach zumindest einem der vorhergehenden Ansprüche **dadurch gekennzeichnet, dass** der Seitenbereich eine Wandstärke (W3) im Bereich 1,2-2,9 mm aufweist.

12. Maskenkissen (1) für eine Vollgesichtsmaske (10) nach zumindest einem der vorhergehenden Ansprüche **dadurch gekennzeichnet, dass** der Seitenbereich eine Wandstärke (W3) im Bereich 1,5-2,5 mm, bevorzugt 2,1 mm +/- 2 mm aufweist.

13. Maskenkissen (1) für eine Vollgesichtsmaske (10) nach zumindest, einem der vorhergehenden Ansprüche **dadurch gekennzeichnet, dass** die Wandstärke W2 von der Basis (4) hin zur Wandstärke W4 der Dichtlippe kontinuierlich abnimmt.

## Claims

1. A mask cushion (1) for a full-face mask (10), having only one sealing lip (2), a sealing area (3) facing the patient's face and leading into the sealing lip, a contact area (4) to the mask body (10), and a deformation area (5) which extends between the contact area (4) and the sealing area (3), the deformation area (5) having a first height (H1) at the bottom at the base (6), a second height (H2) at the top in the nose bridge area (7), and a third height (H3) laterally in the paranasal area (8), with the first height (H1) being at least twice as high as the third height (H3), the mask cushion (1) having a maximum length (L) and a maximum width (B2) and an outer width (B1) in the nose bridge area and a width (B3) in the inner section of the nose area, **characterized in that** the maximum length (L) is 105 mm +/- 6 mm and the maximum width (B2) is 90 mm +/- 4 mm and the outer width in the nose bridge area (B1) being at least 3x, preferably 4x, wider than the width in the inner section of the nose area (B3).

2. The mask cushion (1) for a full-face mask (10) according to Claim 1, **characterized in that** the first height (H1) is greater than the second height (H2), and the second height is greater than the third height (H3).

3. The mask cushion (1) for a full-face mask (10) according to at least one of the preceding claims, **characterized in that** the deformation area (5) at the bottom at the base (6) (H1) is 25 mm +/- 3 mm high and/or at the top in the nose bridge area (7) (H2) is 20 mm +/- 2.5 mm high and/or laterally in the paranasal area (8) (H3) is 11 mm +/- 2 mm.

4. The mask cushion (1) for a full-face mask (10) according to at least one of the preceding claims, **characterized in that** the mask cushion is made of an elastomeric plastic and has at least two different wall thicknesses (9).

5. The mask cushion (1) for a full-face mask (10) according to at least one of the preceding claims, **characterized in that** the wall thickness (9) in the deformation area (5) at the bottom at the base (W2) and at the top in the nose bridge area (W1) is thinner than laterally in the paranasal area (W3).

6. The mask cushion (1) for a full-face mask (10) according to at least one of the preceding claims, **characterized in that** the wall thickness (9) in the deformation area in the nose bridge area (W1) is thinner than the wall thickness in the deformation area (W2) at the base, and the wall thickness (W2) is thinner than laterally in the paranasal area (W3).

7. The mask cushion (1) for a full-face mask (10) according to at least one of the preceding claims, **characterized in that** the wall thickness (W3) is at least twice as thick as the wall thickness (W2).

8. The mask cushion (1) for a full-face mask (10) according to at least one of the preceding claims, **characterized in that** the outer width in the nose bridge area (B1) is 35 mm +/- 3 mm and in the inner section of the nose area (B3) is 8 mm +/-2 mm.

9. The mask cushion (1) for a full-face mask (10) according to at least one of the preceding claims, **characterized in that** the wall thickness (W1) in the nose bridge area (7) is ≤ 0.6 mm.

10. The mask cushion (1) for a full-face mask (10) according to at least one of the preceding claims, **characterized in that** the wall thickness (W1) in the nose bridge area (7) ranges from 0.2 mm to 0.5 mm.

11. The mask cushion (1) for a full-face mask (10) according to at least one of the preceding claims, **characterized in that** the side area has a wall thickness (W3) ranging from 1.2 to 2.9 mm.

12. The mask cushion (1) for a full-face mask (10) according to at least one of the preceding claims, **characterized in that** the side area has a wall thickness (W3) ranging from 1.5 to 2.5 mm, preferably 2.1 mm +/- 2 mm.

13. The mask cushion (1) for a full-face mask (10) according to at least one of the preceding claims, **characterized in that** the wall thickness W2 continuously decreases from the base (4) toward the wall thickness W4 of the sealing lip.

## Revendications

1. **Coussin** de masque (1) destiné à un masque complet (10) avec seulement une lèvre d'étanchéité (2), une zone d'étanchéité (3) tournée vers le visage du patient qui aboutit à la lèvre d'étanchéité, une zone en contact (4) avec la coque du masque (10) et une zone de déformation (5) qui s'étend entre la zone en contact (4) et la zone d'étanchéité (3), dans lequel la zone de déformation (5) présente une première hauteur (H1) en bas à la base (6), une deuxième hauteur (H2) en haut dans la zone du dos du nez (7) et une troisième hauteur (H3) sur les côtés dans la zone latérale du nez (8), dans lequel la première hauteur (H1) est au moins deux fois plus grande que la troisième hauteur (H3), dans lequel le coussin de masque (1) présente une longueur maximale (L) et une largeur maximale (B2) et une largeur (B1) dans la zone du dos du nez à l'extérieur et une largeur (B3) dans la découpe intérieure de la zone du nez, **caractérisé en ce que** la longueur maximale (L) atteint 105 mm +/- 6 mm et la largeur maximale (B2) 90 mm +/- 4 mm et dans lequel la largeur dans la zone du dos du nez à l'extérieur (B1) est au moins 3 fois, de préférence 4 fois plus grande que la largeur dans la découpe intérieure de la zone du nez (B3).

2. Coussin de masque (1) destiné à un masque complet (10) selon la revendication 1, **caractérisé en ce que** la première hauteur (H1) est plus grande que la deuxième hauteur (H2) et que la deuxième hauteur est plus grande que la troisième hauteur (H3).

3. Coussin de masque (1) destiné à un masque complet (10) selon au moins l'une des revendications précédentes, **caractérisé en ce que** la zone de déformation (5) est haute de 25 mm +/- 3 mm (H1) en bas à la base (6) et/ou de 20 mm +/- 2,5 mm (H2) en haut dans la zone du dos du nez (7) et/ou de 11 mm +/- 2 mm (H3) sur les côtés dans la zone latérale du nez (8).

4. Coussin de masque (1) destiné à un masque complet (10) selon au moins l'une des revendications précédentes, **caractérisé en ce que** le coussin de masque est fabriqué à base d'une matière plastique élastomère .et présente au moins deux épaisseurs de paroi différentes (9).

5. Coussin de masque (1) destiné à un masque complet (10) selon au moins l'une des revendications précédentes, **caractérisé en ce que** l'épaisseur de paroi (9) dans la zone de déformation (5) en bas à la base (W2) et en haut dans la zone du dos du nez (W1) est plus mince que sur les côtés dans la zone latérale du nez (W3).

6. Coussin de masque (1) destiné à un masque complet (10) selon au moins l'une des revendications précédentes, **caractérisé en ce que** l'épaisseur de paroi (9) dans la zone de déformation est plus mince dans la zone du dos du nez (W1) que l'épaisseur de paroi dans la zone de déformation à la base (W2) et que l'épaisseur de paroi (W2) est plus mince que sur les côtés dans la zone latérale du nez (W3).

7. Coussin de masque (1) destiné à un masque complet (10) selon au moins l'une des revendications précédentes, **caractérisé en ce que** l'épaisseur de paroi (W3) est au moins le double de l'épaisseur de paroi (W2).

8. Coussin de masque (1) destiné à un masque complet (10) selon au moins l'une des revendications précédentes, **caractérisé en ce que** la largeur atteint 35 mm +/- 3 mm dans la zone du dos du nez à l'extérieur (B1) et 8 mm +/- 2 mm dans la découpe intérieure de la zone du nez (B3).

9. Coussin de masque (1). destiné à un masque complet (10) selon au moins l'une des revendications précédentes, **caractérisé en ce que** l'épaisseur de paroi (W1) est ≤ 0,6 mm dans la zone du dos du nez (7).

10. Coussin de masque (1) destiné à un masque complet (10) selon au moins l'une des revendications précédentes, **caractérisé en ce que** l'épaisseur de paroi (W1) est comprise entre 0,2 et 0,5 mm dans la zone du dos du nez (7).

11. Coussin de masque (1) destiné à un masque complet (10) selon au moins l'une des revendications précédentes, **caractérisé en ce que** la zone latérale présente une épaisseur de paroi (W3) se situant dans la plage 1,2-2,9 mm.

12. Coussin de masque (1) destiné à un masque complet (10) selon au moins, l'une des revendications précédentes, **caractérisé en ce que** la zone latérale présente une épaisseur de paroi (W3) se situant dans la plage 1,5-2,5 mm, de préférence 2,1 mm +/-2 mm.

13. Coussin de masque (1) destiné à un masque complet (10) selon au moins l'une des revendications précédentes, **caractérisé en ce que** l'épaisseur de paroi W2 diminue continuellement de la base (4) jusqu'à l'épaisseur de paroi W4 de la lèvre d'étanchéité.
